# EUROPEAN PATENT APPLICATION

(11) **EP 4 840 406 A1**
(43) Date of publication of application: **18.11.2026**
(21) Application number: 26172779.6
(22) Date of filing: 16.04.2026
(51) Int. Cl.: A61B 10/02

(54) **BRUSH HEAD FOR COLLECTING EXFOLIATIVE CELLS FROM UTERINE T-ZONE**

(30) Priority: 14.05.2025 KR 20250062646
(71) Applicant: BIODYNE CO., LTD., Seoul 04793 (KR)
(72) Inventor: IM, Wook Bin, 06280 Seoul (KR)
(74) Representative: Hoefer & Partner Patentanwälte mbB

(57) **Abstract**

The present invention generally relates to a brush head which is suitable for collecting exfoliative cells from uterine T-zone (Transformation Zone) for cervical cancer screening. In particular, the present invention relates to a brush head configured to effectively collect exfoliative cells from uterine T-zone by means of a two-tier collection brush even when a user inaccurately targets the brush head to the uterine T-zone, and to firmly hold the exfoliative cells during a recovery process by means of a guide brush that is mechanically interlocked with the two-tier collection brush at a periphery thereof. According to the present invention, there is an advantage in that a sufficient quantity of exfoliative cells can be stably obtained from uterine T-zone where transformed cells are mainly located, through which accuracy of cervical cancer screening can be improved.

## Description

### BACKGROUND OF THE INVENTION

### Field of the Invention

The present invention generally relates to a brush head which is suitable for collecting exfoliative cells from uterine T-zone (Transformation Zone) for cervical cancer screening.

In particular, the present invention relates to a brush head configured to effectively collect exfoliative cells from uterine T-zone by means of a two-tier collection brush even when a user inaccurately targets the brush head to the uterine T-zone, and to firmly hold the exfoliative cells during a recovery process by means of a guide brush that is mechanically interlocked with the two-tier collection brush at a periphery thereof.

### Description of the Related Art

Generally, cervical cancers include adenocarcinoma and squamous cell carcinoma, and their ratios are approximately 5 percent and 95 percent, respectively. For cervical cancer screening, a method of collecting exfoliative cells from the subject's uterus and observing them under a microscope is widely used.

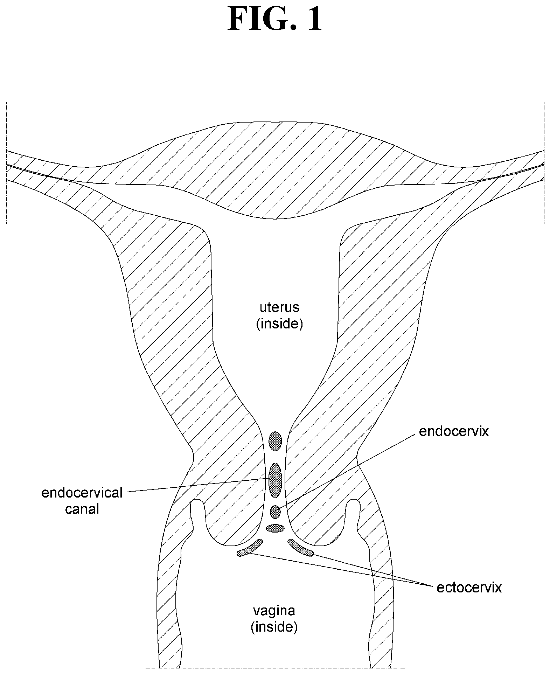

FIG. 1 is a drawing briefly illustrating the structure of the inside of the uterus. Referring to FIG. 1, as stimulation or inflammation is repeated, squamous cells of the ectocervix move into the endocervix, and a phenomenon occurs in which glandular cells of the endocervix transform into a form of metaplastic squamous cells. The phenomenon in which glandular cells change into squamous cells is called squamous metaplasia. At this time, if an abnormality occurs in the cell nucleus, it is called dysplasia, and if cell transformation further proceeds, it becomes carcinoma.

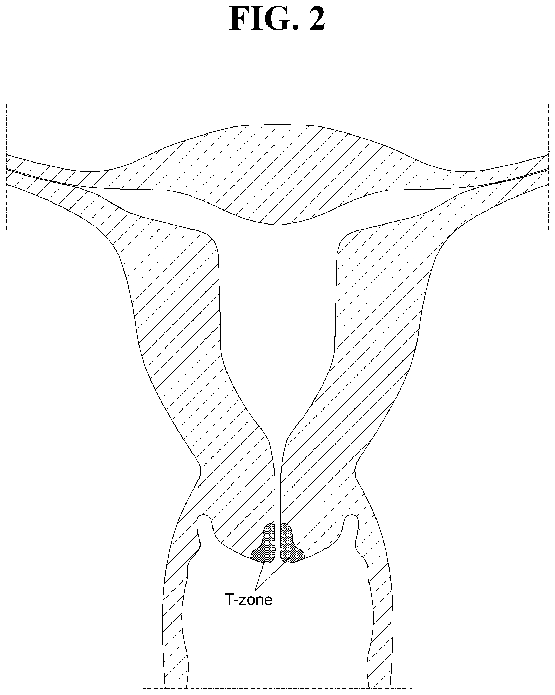

An area in which the cell transformation as described above occurs is generally called a 'T-zone' (Transformation Zone, T-zone). To increase the accuracy of cervical cancer screening, it is important to collect exfoliative cells from the T-zone. If exfoliative cells are collected from other areas, it is impossible to determine whether cell transformation has occurred. The T-zone is approximately the entrance where the endocervical canal begins in FIG. 1 and tends to gradually move into the endocervical canal as one gets older. FIG. 2 is a drawing schematically illustrating the location of the uterine T-zone.

In a situation where a user visits an examination institution for cervical cancer screening, exfoliative cells may be collected from the uterine T-zone. On the other hand, in a situation where the user self-collects exfoliative cells for sending them to an examination institution (e.g., a hospital) with putting them in a vial, it is very difficult to accurately target the uterine T-zone. Furthermore, even in an examination institution, if a collector lowers his attention, the uterine T-zone may be inaccurately targeted. This is because the size of the uterine T-zone is actually very small. If exfoliative cells are collected in such an inaccurately targeted state, there is a problem that the cervical cancer screening result becomes inaccurate.

Accordingly, mechanical improvement of an exfoliative cell collection apparatus is desired to assist in stably collecting a sufficient quantity of exfoliative cells from the uterine T-zone even in a situation where the uterine T-zone is inaccurately targeted. In particular, mechanical improvement of an exfoliative cell collection apparatus is desired to assist in firmly holding the once-collected exfoliative cells without missing them by spilling them to the outside during a collection apparatus recovery process.

### SUMMARY OF THE INVENTION

It is an object of the present invention to provide a brush head which is suitable for collecting exfoliative cells from uterine T-zone (Transformation Zone) for cervical cancer screening.

In particular, it is an object of the present invention to provide a brush head configured to effectively collect exfoliative cells from uterine T-zone by means of a two-tier collection brush even when a user inaccurately targets the brush head to the uterine T-zone, and to firmly hold the exfoliative cells during a recovery process by means of a guide brush that is mechanically interlocked with the two-tier collection brush at a periphery thereof.

To achieve the object, the present invention proposes A brush head for collecting exfoliative cells from uterine T-zone (Transformation Zone), having a configuration capable of stably collecting exfoliative cells from the uterine T-zone and trapping the exfoliative cells such that the exfoliative cells do not flow out to the outside during a recovery process even in a state where a user inaccurately targets the uterine T-zone to self-collect exfoliative cells in the user's uterus for cervical cancer screening.

The brush head according to the present invention may comprise: a support rod 110 of a flexible material forming a base pillar; a first collection brush 120 having a plurality of first probe strands 121 of a flexible material that are formed to protrude forward in a state of being adjacent to each other at a center of a tip end portion of the support rod 110 and are flexibly bent by an external force; a second collection brush 140 having a plurality of second probe strands 141 of a flexible material that are formed to protrude forward with a relatively smaller height than that of the first probe strands 121 in a state of being adjacent to each other in parallel with the first probe strands 121 in a form of surrounding the first probe strands 121 along a peripheral outer circumferential direction of the first collection brush 120 at the tip end portion of the support rod 110; and a guide brush 130 having a plurality of guide leaves 131 whose one ends are sequentially connected to a tip end edge portion of the support rod 110 along a circumferential direction of the first collection brush 120 and the second collection brush 140, whose other ends are formed to extend obliquely toward a tip end portion of the first collection brush 120, that are expanded in a direction away from the first collection brush 120 and the second collection brush 140 by elastic resilience in a process of emerging from an external housing member 300 to collect exfoliative cells from the uterine T-zone, and that are constricted in a form of surrounding the first collection brush 120 and the second collection brush 140 by being pressed by an inner wall of the housing member 300 in a process of entering the inside of the housing member 300 to recover the brush head from which the exfoliative cells are collected.

In the present invention, the plurality of first probe strands 121 and the plurality of second probe strands 141 constitute a collection brush of a two-tier structure for collecting exfoliative cells from the uterine T-zone. When the support rod 110 is advanced forward by a user operation in a state where the user inaccurately targets the brush head to the uterine T-zone during an exfoliative cell collection process, by the collection brush of the two-tier structure, the first probe strands 121 having the relatively large height in the collection brush of the two-tier structure primarily contact an ectocervix near the uterine T-zone in the uterus and are flexibly bent toward a direction opposite to the uterine T-zone by a shape of the ectocervix, so that the second probe strands 141 having the relatively small height, which are located at a position opposite to a bending direction of the first probe strands 121 in the collection brush of the two-tier structure, contact the uterine T-zone to collect exfoliative cells at a uterine T-zone point.

### BRIEF DESCRIPTION OF THE DRAWINGS

FIG. 1 is a drawing briefly illustrating the structure of the inside of the uterus.
FIG. 2 is a drawing schematically illustrating the location of the uterine T-zone.
FIG. 3 is a drawing illustrating the overall configuration of an exfoliative cell collection apparatus in the present invention.
FIG. 4 is a drawing illustrating a state in which the brush head partially advances forward of the housing member.
FIG. 5 is a drawing illustrating a state in which the brush head completely advances forward of the housing member.
FIG. 6 is a drawing illustrating the overall configuration of the brush head according to the present invention.
FIG. 7 is a drawing illustrating the brush head according to the present invention viewed from above.
FIG. 8 is a drawing illustrating a collection brush of a two-tier structure in the present invention
FIG. 9 is an enlarged view of the two-tier structure portion in the present invention.
FIG. 10 is an exemplary view in a case where the exfoliative cell collection apparatus is accurately targeted to a uterine T-zone in the present invention.
FIG. 11 is an exemplary view of exfoliative cell collection in an accurate targeting situation in the present invention.
FIG. 12 is an exemplary view in a case where the exfoliative cell collection apparatus is inaccurately targeted to the uterine T-zone in the present invention.
FIG. 13 is an exemplary view in which the first collection brush begins to contact the ectocervix in an inaccurate targeting situation.
FIG. 14 is an exemplary view in which the second collection brush begins to contact the ectocervix in an inaccurate targeting situation.
FIG. 15 is an exemplary view of exfoliative cell collection in an inaccurate targeting situation.

### DESCRIPTION OF EMBODIMENTS

Hereinafter, the present invention will be described in detail with reference to the drawings.

The present invention generally relates to a brush head which is suitable for collecting exfoliative cells from uterine T-zone (Transformation Zone) for cervical cancer screening. In particular, a brush head is proposed that enables stable collection of a sufficient quantity of exfoliative cells from the uterine T-zone even in a situation where targeting to the uterine T-zone is inaccurate.

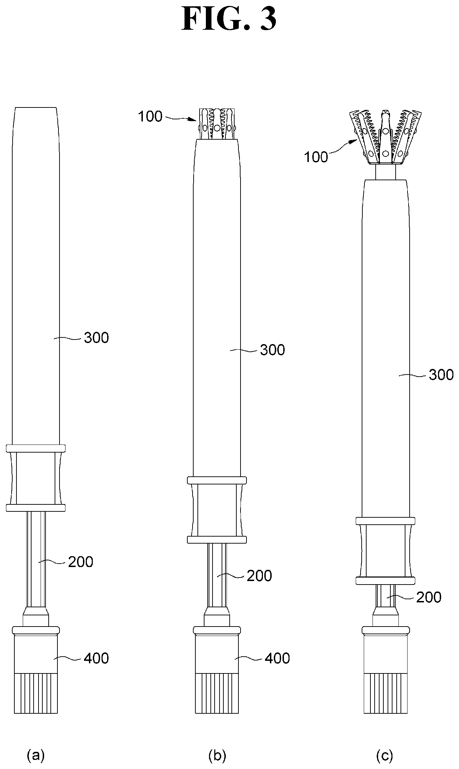

FIG. 3 is a drawing illustrating the overall configuration of an exfoliative cell collection apparatus in the present invention.

Referring to FIG. 3, the exfoliative cell collection apparatus may include a brush head 100, a piston rod 200, a housing member 300, and a handle member 400.

The brush head 100 is a component for collecting exfoliative cells by contacting a uterine T-zone of a human body according to a user's operation.

The piston rod 200 is a component that is connected to the rear of the brush head 100 inside the housing member 300 and guides the forward and backward movement of the brush head 100 in response to a user's operation.

The housing member 300 is a hollow component, such as a cylindrical shape, to provide a space in which other components can be contained. The housing member 300 provides a space so that the brush head 100 can emerge or retract at a tip end thereof when the piston rod 200 moves in the forward and backward direction by user operation while the brush head 100 and the piston rod 200 are inserted inside.

The handle member 400 is a component that is connected to the rear end of the piston rod 200 and is disposed outside the rear of the housing member 300 to assist in user operation.

First, a process of collecting exfoliative cells from the subject's body will be described.

As shown in FIG. 3 (a), the tip end of the housing member 300 is moved into the inside of the vagina of the user (subject) while the brush head is constricted and accommodated inside the housing member 300.

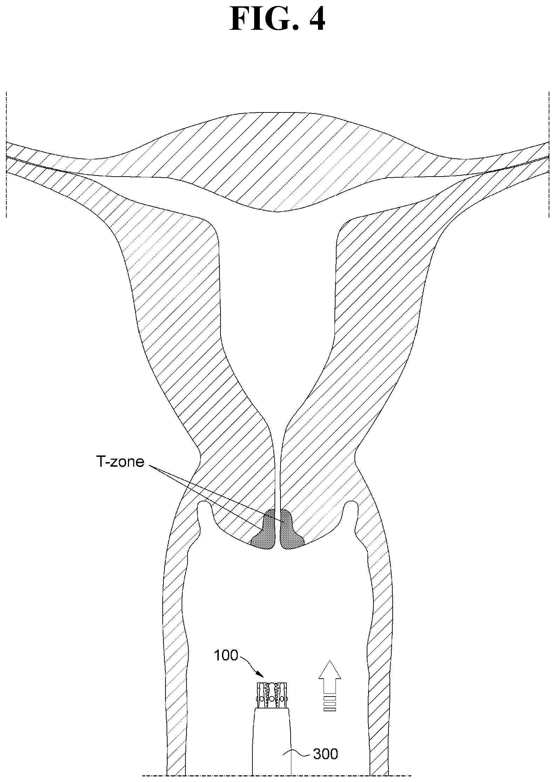

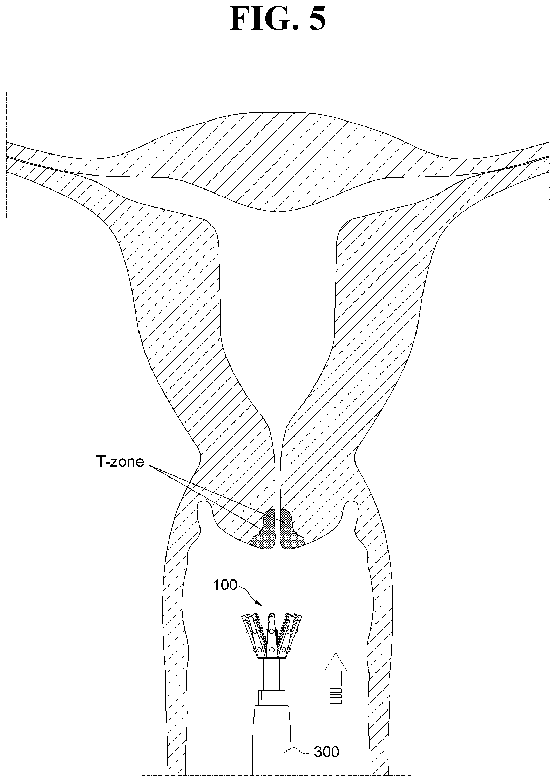

When it is determined that the housing member 300 has been sufficiently inserted, the user pushes the piston rod 200 while gripping the housing member 300 to advance the brush head 100 forward. The brush head 100 advances forward of the housing member 300 as shown in FIG. 3 (c) via FIG. 3 (b). At this time, the brush head 100 is expanded by the elastic resilience of the material to approximately 15 degrees to 25 degrees. FIG. 4 is a drawing illustrating a state in which the brush head 100 partially advances forward of the housing member 300 in the present invention, and FIG. 5 is a drawing illustrating a state in which the brush head 100 completely advances forward of the housing member 300 in the present invention.

The user further moves the exfoliative cell collection apparatus forward to make the brush head 100 reach the uterine T-zone, and then rubs the brush head 100 against the inner wall of the cervix so that the exfoliative cells are collected.

Next, a process of recovering exfoliative cells to the outside of the subject's body will be described.

The user pulls the piston rod 200 backward while gripping the housing member 300. Accordingly, the brush head 100 gradually enters the inside of the housing member 300 while being constricted as shown in FIG. 3 (a) from the state of FIG. 3 (c) via FIG. 3 (b). Subsequently, the user moves the housing member 300 backward to remove the brush head 100 from the subject's body.

Then, although not shown in FIG. 3, the user mechanically detaches the brush head 100 by further pulling the piston rod 200 backward while gripping and fixing the housing member 300. Then, the detached brush head 100 is put into a vial (not shown) and sent to an examination institution (e.g., a hospital). The examination institution performs cervical cancer screening using the exfoliative cells attached to the brush head 100.

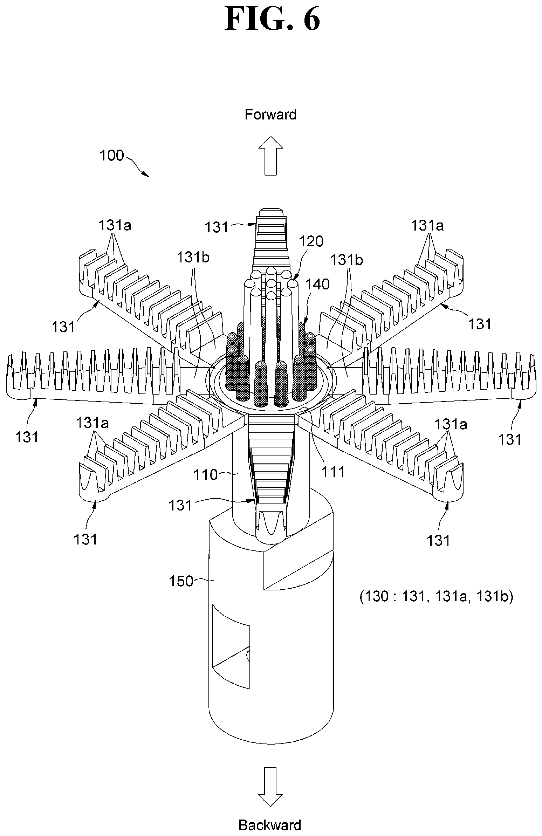

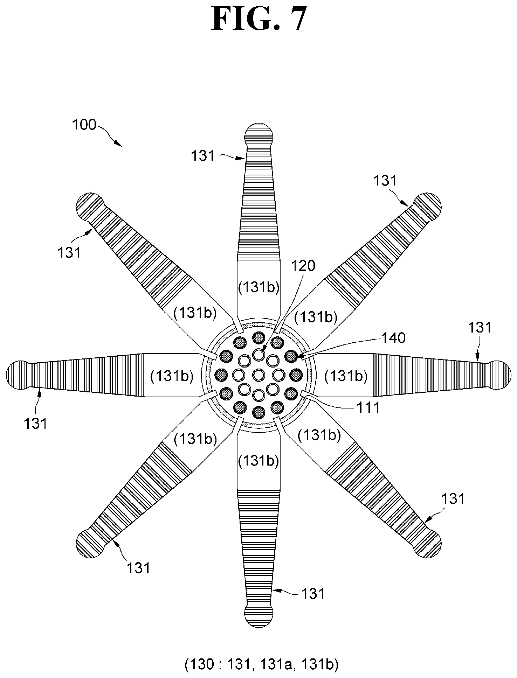

FIG. 6 is a drawing illustrating the overall configuration of the brush head 100 according to the present invention, and FIG. 7 is a drawing illustrating the brush head 100 according to the present invention viewed from above. In FIGS. 6 and 7, for convenience, the brush head 100 is shown in a fully expanded state.

The brush head 100 according to the present invention includes a support rod 110, a first collection brush 120, a guide brush 130, and a second collection brush 140. These components 110, 120, 130, and 140 may be made of a medical synthetic resin material having flexible properties, for example, medical elastomers which are medical polymer materials. In addition, these components 110, 120, 130, and 140 may preferably be integrally formed.

The support rod 110 is a component that forms a base pillar of the brush head 100 and allows the brush head 100 to move forward or backward in response to a user operation on the piston rod 200.

The first collection brush 120 is formed by a plurality of first probe strands 121 protruding forward in an adjacent state at a center of a tip end portion of the support rod 110. The first collection brush 120 is composed of a bundle of first probe strands 121. The first probe strands 121 are made of a flexible medical synthetic resin material and can be flexibly bent.

The second collection brush 140 is formed by a plurality of second probe strands 141 protruding forward in an adjacent state and in parallel with the first probe strands 121 in an outer region of the tip end portion of the support rod 110. The second collection brush 140 is composed of a bundle of second probe strands 141. The second collection brush 140 may be formed along a peripheral circumferential direction of the first collection brush 120 in an area corresponding to between the first collection brush 120 and the guide brush 130. The second probe strands 141 are made of a flexible medical synthetic resin material and can be flexibly bent.

The guide brush 130 is a component that includes a plurality of guide leaves 131 in the form of wings and surrounds the first collection brush 120 and the second collection brush 140. The guide brush 130 is formed along the circumferential direction of the first collection brush 120 and the second collection brush 140 at the edge portion of the tip end portion of the support rod 110. One ends of the guide leaves 131 are sequentially connected so as to surround the first collection brush 120 and the second collection brush 140. The other ends of the guide leaves 131 may be formed to extend obliquely toward the tip end portions of the first and second collection brushes 120 and 140. When the brush head 100 enters the housing member 300 as shown in FIG. 3 (a), the guide brush 130 is pressed by the inner wall of the housing member 300 and is constricted to surround the first and second collection brushes 120 and 140. Conversely, when the brush head 100 emerges from the housing member 300 as shown in FIG. 3 (c), the guide leaves 131 expand in a direction away from the first and second collection brushes 120 and 140 due to elastic resilience between the components 120, 130, and 140 that were in contact with each other.

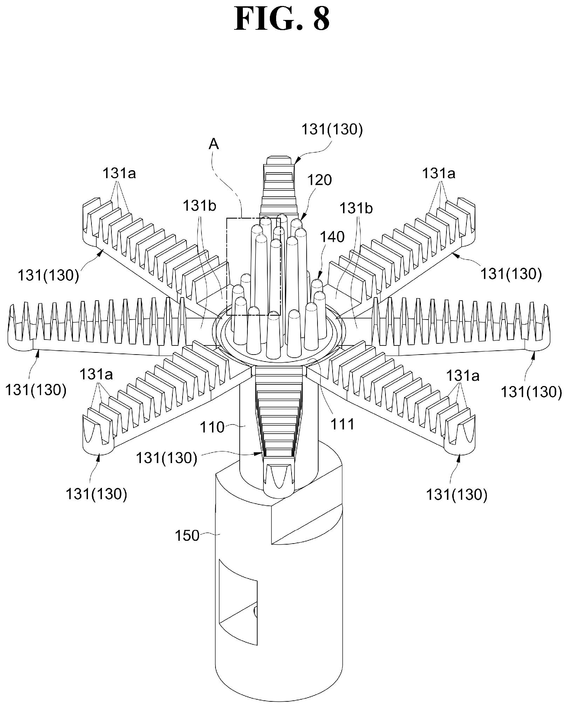

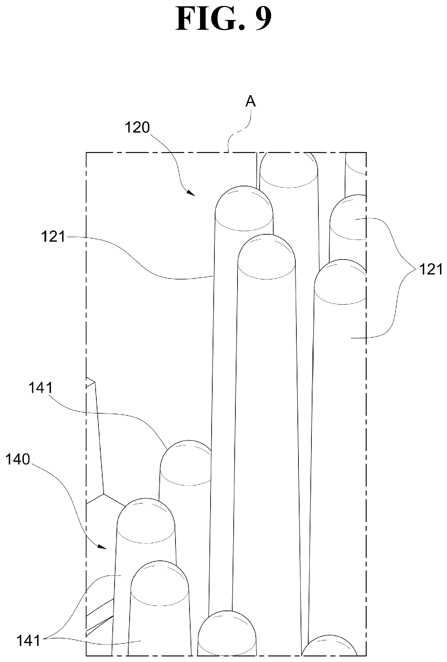

FIG. 8 is a drawing illustrating a collection brush of a two-tier structure in the present invention, and FIG. 9 is an enlarged view of the two-tier structure portion in the present invention.

The present invention is characterized in that the collection brush is configured in a two-tier structure. The first collection brush 120 is composed of a bundle of first probe strands 121 having a relatively large height and is disposed in a central region of the collection brush. The second collection brush 140 is composed of a bundle of second probe strands 141 having a relatively small height and is disposed at the periphery of the first collection brush 120. In one embodiment, the height of the first collection brush 120 is at least twice that of the second collection brush 140.

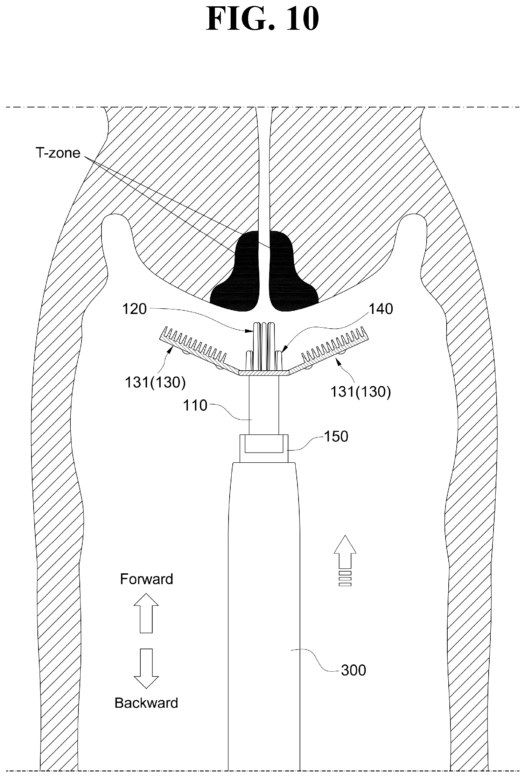

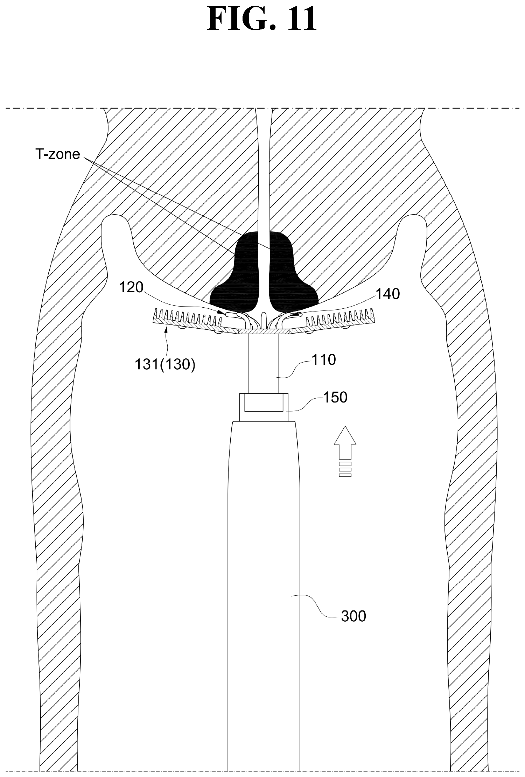

FIG. 10 is an exemplary view in a case where the exfoliative cell collection apparatus is accurately targeted to a uterine T-zone in the present invention, and FIG. 11 is an exemplary view of exfoliative cell collection in an accurate targeting situation in the present invention.

When the user accurately targets the exfoliative cell collection apparatus to the uterine T-zone, the first collection brush 120 and the second collection brush 140 can collect a sufficient quantity of exfoliative cells from the uterine T-zone.

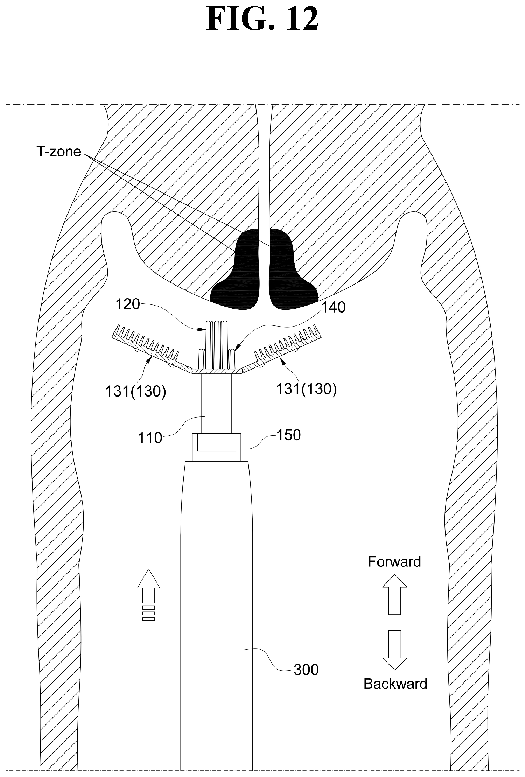

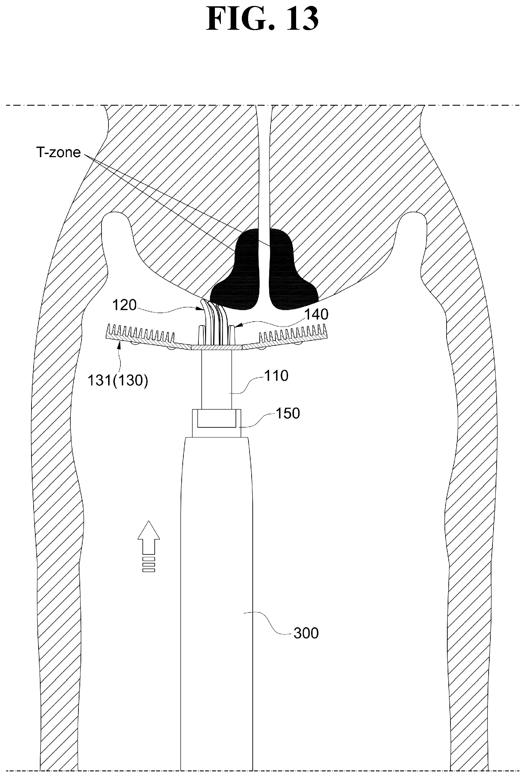

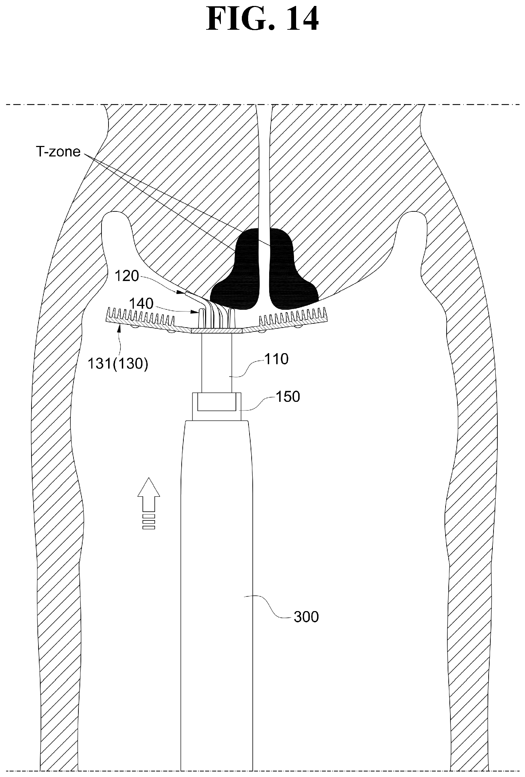

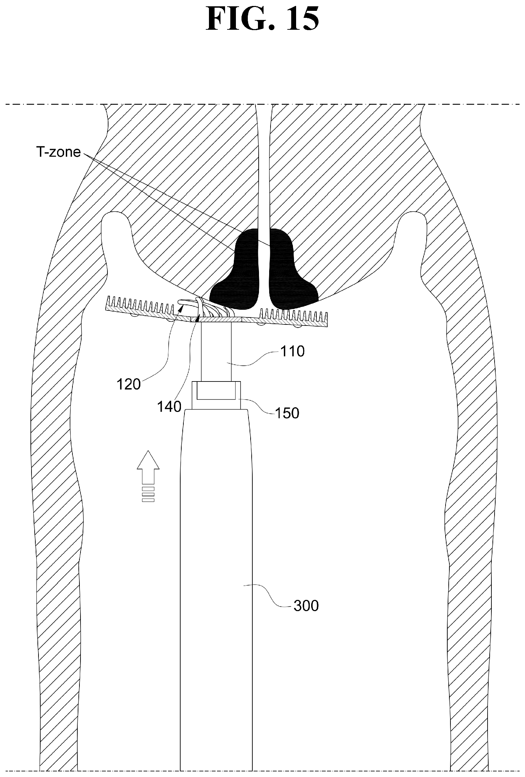

FIG. 12 is an exemplary view in a case where the exfoliative cell collection apparatus is inaccurately targeted to the uterine T-zone in the present invention, and FIGS. 13 to 15 are exemplary views of an exfoliative cell collection process in an inaccurate targeting situation in the present invention. Specifically, FIG. 13 is an exemplary view in which the first collection brush 120 begins to contact the ectocervix in an inaccurate targeting situation, FIG. 14 is an exemplary view in which the second collection brush 140 begins to contact the ectocervix in an inaccurate targeting situation, and FIG. 15 is an exemplary view of exfoliative cell collection in an inaccurate targeting situation.

When collecting exfoliative cells for cervical cancer screening, situations may occur in which the exfoliative cell collection apparatus is inaccurately targeted to the uterine T-zone. For example, in a situation where exfoliative cells are self-collected, accurate targeting to the uterine T-zone is very difficult. Even in a situation where a doctor collects exfoliative cells, inaccurate targeting can occur if attention is lowered.

In such an inaccurate targeting situation, the first collection brush 120 having a relatively large height reaches the ectocervix at the periphery of the uterine T-zone as shown in FIG. 13. As the user pushes the piston rod 200 or the housing member 300 forward, the first collection brush 120 presses the ectocervix, and an external force is applied to the first collection brush 120 due to a reaction thereto. Due to this external force, as shown in FIGS. 13 and 14, the first probe strands 121 of the first collection brush 120 are softly bent toward a direction opposite to the uterine T-zone along the outer surface of the ectocervix.

When the first collection brush 120 is sufficiently bent, as shown in FIGS. 14 and 15, the second collection brush 140 having a relatively small height, located in a direction opposite to the direction to which the first collection brush 120 is bent, comes into contact with the uterine T-zone area. Accordingly, the second collection brush 140 can collect a sufficient quantity of exfoliative cells from the uterine T-zone area.

To enable such an operation, in the present invention, the first collection brush 120 is formed to have a large height in the center, whereas the second collection brush 140 is configured to have a relatively small height while surrounding the periphery thereof. Due to this two-tier structure of collection brush, exfoliative cells at the uterine T-zone point can be effectively collected even when a user inaccurately targets the exfoliative cell collection apparatus to the uterine T-zone.

Meanwhile, the guide leaves 131 of the guide brush 130 may form flat inward-facing surfaces in a direction facing the first and second collection brushes 120 and 140 as shown in FIGS. 6 and 7. A plurality of horizontal protrusions 131a are formed on the inward-facing surface, in which the horizontal protrusions 131a protrude in multiple steps toward the first and second collection brushes 120 and 140. These horizontal protrusions 131a may be formed in a blade pattern along the circumferential direction of the first and second collection brushes 120 and 140.

The horizontal protrusions 131a may be formed on the entire inward-facing surface. However, it is preferable that the horizontal protrusions 131a have a protrusion-free region 131b where a predetermined area is recessed or empty from the bottom of the inward-facing surface. Referring to FIGS. 6 and 7, it can be seen that the horizontal protrusions 131a are not formed at the bottom of the inward-facing surface of the guide leaves 131.

The protrusion-free region 131b is preferably formed to correspond to, and more preferably to perfectly engage with, the height of the second collection brush 140. By having the protrusion-free region 13 lb as described above, when the guide brush 130 is constricted to surround the first and second collection brushes 120 and 140 as shown in FIGS. 3 (a) and (b), the horizontal protrusions 131a, the first probe strands 121, and the second probe strands 141 can be mechanically interlocked and inter-engaged with each other. This structure enables the exfoliative cells to be firmly held without missing them during the recovery process of the brush head 100 from the subject's body after collecting the exfoliative cells. This makes it possible to stably obtain a sufficient quantity of exfoliative cells from the uterine T-zone, thereby improving the accuracy of cervical cancer screening.

When recovering the brush head 100 from the subject's body after collecting the exfoliative cells, the volume of the brush head 100 is made small by causing it to be constricted as in FIG. 3 (b) from the expanded state of FIG. 3 (c). During this process, the guide brush 130 is constricted in a form of surrounding the first and second collection brushes 120 and 140. Then, the brush head 100 is inserted into the housing member 300 as shown in FIG. 3 (a), and the exfoliative cell collection apparatus is removed from the subject's body.

When collecting exfoliative cells in the uterine T-zone area, the exfoliative cells are mixed with liquid medium and attached to the first probe strands 121 and the second probe strands 141 in a clumped state. That is, immediately after collecting the exfoliative cells, the brush head 100 is in a state in which the exfoliative cells and the liquid medium (body fluid) are heavily attached.

In the process of recovering the brush head 100, if the brush head 100 is constricted from the state of FIG. 3 (c) to the state of FIG. 3 (b), an action of squeezing out the body fluid toward the outside of the brush head 100 is formed. In order to prevent the exfoliative cells from flowing out and being lost together with the body fluid during this process, the present invention adopts a configuration in which the two-tier collection brushes 120 and 140 and the guide brush 130 are mechanically interlocked. The exfoliative cells are mechanically and firmly trapped between the two-tier collection brushes 120 and 140 and the horizontal protrusions 131a and the protrusion-free region 131b of the guide brush 130, and only the body fluid escapes therebetween.

On the other hand, the support rod 110 may include a folding groove member 111. The folding groove member 111 is formed in an annular shape recessed along the outer peripheral direction of the second collection brush 140 in a tip end region corresponding to between the guide brush 130 and the second collection brush 140, as shown in FIGS. 6 and 7. The recessed shape of the folding groove member 111 is well illustrated in FIG. 11. The folding groove member 111 guides the folding operation of the guide leaves 131. When the guide brush 130 is constricted to surround the first collection brush 120 as shown in FIG. 3 (b), or conversely, when it is expanded outward as shown in FIG. 3 (c), the folding groove member 111 guides the guide leaves 131 to fold or unfold in an accurate shape and direction.

On the other hand, the brush head 100 may further include a grip block 150 as shown in FIG. 6. The grip block 150 may preferably be made of a flexible synthetic resin material, and is integrally connected to the rear end of the support rod 110 and can be detachably connected to the tip end of the piston rod 200.

The structure of the brush head 100 of the present invention described above is designed to stably obtain a sufficient quantity of exfoliative cells from the uterine T-zone where transformed cells are mainly located, thereby increasing the accuracy of cervical cancer screening.

According to the present invention, by forming a collection brush in a two-tier structure, there is an advantage in that exfoliative cells can be effectively collected from uterine T-zone even in a situation where a user inaccurately targets an exfoliative cell collection apparatus to the uterine T-zone.

In addition, according to the present invention, as a guide brush and a collection brush are mechanically interlocked with each other, there is also an advantage of firmly holding exfoliative cells during a collection apparatus recovery process.

Due to the advantages of the present invention as described above, a sufficient quantity of exfoliative cells can be stably obtained from uterine T-zone where transformed cells are mainly located, through which accuracy of cervical cancer screening can be improved.

While the present invention has been described with respect to the specific embodiments, it will be apparent to those skilled in the art that various changes and modifications may be made without departing from the spirit and scope of the invention as defined in the following claims.

## Claims

1. A brush head for collecting exfoliative cells from uterine T-zone (Transformation Zone), comprising:
a support rod of a flexible material forming a base pillar;
a first collection brush having a plurality of first probe strands of a flexible material that are formed to protrude forward in a state of being adjacent to each other at a center of a tip end portion of the support rod and are flexibly bent by an external force;
a second collection brush having a plurality of second probe strands of a flexible material that are formed to protrude forward with a relatively smaller height than that of the first probe strands in a state of being adjacent to each other in parallel with the first probe strands in a form of surrounding the first probe strands along a peripheral outer circumferential direction of the first collection brush at the tip end portion of the support rod; and
a guide brush having a plurality of guide leaves whose one ends are sequentially connected to a tip end edge portion of the support rod along a circumferential direction of the first collection brush and the second collection brush, whose other ends are formed to extend obliquely toward a tip end portion of the first collection brush, that are expanded in a direction away from the first collection brush and the second collection brush by elastic resilience in a process of emerging from an external housing member to collect exfoliative cells from the uterine T-zone, and that are constricted in a form of surrounding the first collection brush and the second collection brush by being pressed by an inner wall of the housing member in a process of entering the inside of the housing member to recover the brush head from which the exfoliative cells are collected,
wherein the plurality of first probe strands made of a flexible material and formed in the central portion with a relatively large height and the plurality of second probe strands made of a flexible material and formed in a peripheral portion with the relatively small height constitute a collection brush of a two-tier structure for collecting exfoliative cells from the uterine T-zone,
and wherein the brush head is configured such that when the support rod is advanced forward by a user operation in a state where the user inaccurately targets the brush head to the uterine T-zone during an exfoliative cell collection process, by the collection brush of the two-tier structure, the first probe strands having the relatively large height in the collection brush of the two-tier structure primarily contact an ectocervix near the uterine T-zone in the uterus and are flexibly bent toward a direction opposite to the uterine T-zone by a shape of the ectocervix, so that the second probe strands having the relatively small height, which are located at a position opposite to a bending direction of the first probe strands in the collection brush of the two-tier structure, contact the uterine T-zone to collect exfoliative cells at a uterine T-zone point.

2. The brush head according to claim 1,
wherein the guide leaf forms a flat inward-facing surface toward the first collection brush and the second collection brush,
wherein the guide leaf includes: a plurality of horizontal protrusions formed on the inward-facing surface to protrude in multiple steps toward the first collection brush; and a protrusion-free region, in which the horizontal protrusions are not formed so that a predetermined area is recessed or empty from a bottom of the inward-facing surface, to be engaged with a height of the second collection brush in the collection brush of the two-tier structure,
and wherein the plurality of first probe strands and the plurality of second probe strands constituting the collection brush of the two-tier structure are configured to be mechanically interlocked and inter-engaged with the plurality of horizontal protrusions and the protrusion-free region, so that exfoliative cells attached to the first probe strands and the second probe strands in a state of being mixed with a liquid medium in a process of recovering the brush head from a subject's body after collecting the exfoliative cells are mechanically trapped between the collection brush of the two-tier structure, the horizontal protrusions, and the protrusion-free region.

3. The brush head according to claim 2,
wherein the support rod includes a circular folding groove member recessed along an outer peripheral direction of the second collection brush in a tip end region corresponding to between the guide brush and the second collection brush, so as to be configured to guide a folding operation of the plurality of guide leaves when the guide brush is constricted to surround the first collection brush.

4. The brush head according to claim 3,
wherein the horizontal protrusions are formed in a blade pattern along a circumferential direction of the second collection brush.
